# EUROPÄISCHE PATENTSCHRIFT

(11) **EP 1 942 792 B1**
(45) Veröffentlichungstag und Bekanntmachung des Hinweises auf die Patenterteilung: **07.03.2012**
(21) Anmeldenummer: 06805460.0
(22) Anmeldetag: 18.10.2006
(51) Int. Cl.: A61B 5/00, A61B 5/107

(54) **MESSVORRICHTUNG UND VERFAHREN ZUR BESTIMMUNG DES AKTIVITÄTSSTATUS INITIALKARIÖSER SCHMELZLÄSIONEN**
MEASURING DEVICE AND METHOD FOR DETERMINING THE ACTIVITY STATUS OF INITIAL DECAYED ENAMEL LESIONS
DISPOSITIF DE MESURE ET PROCEDE DE DETERMINATION DE L'ETAT D'ACTIVITE DE LESIONS CARIEUSES INITIALES DE L'EMAIL

(30) Priorität: 26.10.2005 DE 102005052294
(43) Veröffentlichungstag der Anmeldung: 16.07.2008
(73) Patentinhaber: Jaruszewski, Lutz, 31141 Hildesheim (DE)
(72) Erfinder: Jaruszewski, Lutz, 31141 Hildesheim (DE)
(74) Vertreter: Freitag, Joachim
(86) Internationale Anmeldenummer: PCT/DE2006/001861
(87) Internationale Veröffentlichungsnummer: WO 2007/065389

(56) Entgegenhaltungen:
- WO-A-99/01746
- DE-A1- 19 640 495
- US-A- 5 785 651
- US-A1- 2005 122 518
- ROUSSEAU C ET AL: "Application of a novel confocal imaging technique for early the detection of dental decay" PROCEEDINGS OF THE SPIE - THE INTERNATIONAL SOCIETY FOR OPTICAL ENGINEERING SPIE-INT. SOC. OPT. ENG USA, Bd. 4610, 2002, Seiten 92-99, XP002449684 ISSN: 0277-786X
- YAMADA MAGDA KIYOKO ET AL: "Imaging and non-contact profile analysis of Nd:YAG laser-irradiated teeth by scanning electron microscopy and confocal laser scanning microscopy." DENTAL MATERIALS JOURNAL DEC 2003, Bd. 22, Nr. 4, Dezember 2003 (2003-12), Seiten 556-568, XP008083166 ISSN: 0287-4547
- CHA S ET AL: "NONTRANSLATIONAL THREE-DIMENSIONAL PROFILOMETRY BY CHROMATIC CONFOCAL MICROSCOPY WITH DYNAMICALLY CONFIGURABLE MICROMIRROR SCANNING" APPLIED OPTICS, OSA, OPTICAL SOCIETY OF AMERICA, WASHINGTON, DC, US, Bd. 39, Nr. 16, 1. Juni 2000 (2000-06-01), Seiten 2605-2613, XP000951755 ISSN: 0003-6935
- J GARZON, J MENESES, G TRIBILLON, T GHARBI, A PLATA: "Chromatic confocal microscopy by means of continuum light generated through a standard single-mode fibre" JOURNAL OF OPTICS A: PURE AND APPLIED OPTICS, 23. April 2004 (2004-04-23), Seiten 544-548, XP002449685 UK
- JUSKAITIS R ET AL: "Confocal microscopy using optical fibre imaging bundles" PROCEEDINGS OF THE SPIE - THE INTERNATIONAL SOCIETY FOR OPTICAL ENGINEERING SPIE-INT. SOC. OPT. ENG USA, Bd. 2655, 1996, Seiten 92-94, XP002449687 ISSN: 0277-786X

## Beschreibung

Die Erfindung bezieht sich auf eine Messvorrichtung und ein Verfahren zur Bestimmung des Aktivitätsstatus initialkariöser Schmelzläsionen. Durch Punktmessungen mittels eines optischen Handsensors können außerdem Erosionen und weitere Oberflächenveränderungen festgestellt werden.

Karies ist eine bakteriell induzierte, sich langsam entwickelnde Erkrankung in den Zahnhartgeweben. Sie wird durch kariogene Plaque verursacht, in der bestimmte Mikroorganismen schwache Säuren produzieren, während sie Kohlenhydrate metabolisieren. Die Säuren lösen allmählich das unter der Plaque liegende Mineral auf, sowohl das oberflächliche, als auch das tiefer unter der Oberfläche gelegene, wodurch irreversible strukturelle Veränderungen in den Zahnhartgeweben verursacht werden (Ekstrand KR, J Dent Res 83 (Spec Iss C): C67-C71, 2004).

Entsprechend einer heute gültigen Zoneneinteilung der Schmelzkaries nach Silverstone LM, Johnson NW, Hardie JM, Williams RDA: Dental Caries, Aetiology, Pathology and Prevention, Macmillan Press Ltd., London 1981 bilden sich bei akutem Verlauf opake Läsionen (white spot), die bei rechtzeitiger Diagnose, Mundhygiene, kontrollierter Ernährung und Fluoridierung wieder remineralisieren können. Eine superfizielle Demineralisation führt zur Auflösung der Prismenperipherie oder der Prismenkerne und über diese Wege zum subfiziellen Defekt. Die Demineralisation und Remineralisation erfolgt über elektronenmikroskopisch nachweisbare Poren und Mikrokanäle mit einem Durchmesser von 10 - 20 µm in der Oberflächenzone, die für den lonenaustausch von entscheidender Bedeutung sind (Haikel Y, Frank RM, Voegel JC, Caries Research 1983; 17: 1-13). Während des kariösen Angriffs, bei dem Calcium- und Phosphationen aus Speichel und Plaquefluid und aus dem Körper der Läsion verwendet werden, entsteht eine als Remineralisationsprodukt ausgebildete Oberflächenzone.

Das moderne Verständnis der dynamischen Natur kariöser Prozesse, wonach eine Progression in jedem Stadium arretiert werden kann, unterstützt die Wichtigkeit der klinischen Einschätzung des kariösen Aktivitätsstatus. Sie hängt von der Fähigkeit ab, feine Veränderungen im Schmelz durch visuelle und taktile Inspektionen zu identifizieren.

Eine exakte und zuverlässige Bewertung der Kariesaktivität, das heißt eine Bewertung der Geschwindigkeit, mit der sich der kariöse Prozess im Laufe der Zeit fortsetzen wird, ist somit von entscheidender Bedeutung für die Kariesrisikoeinschätzung und für einen korrekten Behandlungsplan für die kariöse Erkrankung.

Von Ekstrand KR u. Mit., Caries Research 2005; 39; 173-177) wird die Meinung vertreten, dass Zahnärzte nicht in der Lage sind, anhand der visuellen oder taktilen Erscheinung von initialkariösen Läsionen deren Aktivität oder Inaktivität zuverlässig und reproduzierbar zu beurteilen.

Zu den bisher üblichen und klinisch anwendbaren instrumentellen Untersuchungsmethoden zählen neben der Bissflügel-Röntgendiagnostik und der Transillumination zum Beispiel die quantitative lichtinduzierte Fluoreszenz (QLF) und die Laserfluoreszenz (LF).

Bekannte Vorrichtungen und Verfahren zur Laserfluoreszenz sind z. B. in DE 93 17 984 U1, DE 42 00 741 A1 und in DE 197 09 500 C1 beschrieben.

Die quantitative lichtinduzierte Fluoreszenz erlaubt auf der Grundlage von Fluoreszenzbildern der Eigenfluoreszenz des Schmelzes Aussagen über die Größe und den Mineralverlust von Läsionen (Tranaeus S, Heinrich-Weltzien R, Kühnisch J, Stösser L, Angmar-Mansson B, Medical Laser Application (2001)).

Bei der Laserfluoreszenz kommt es nach Anregung mit rotem Licht hauptsächlich zur Fluoreszenz in der Karies. Die hierbei fluoreszierenden Porphyrine werden von den eingewanderten Bakterien produziert (Hibst R, Paulus R, Lussi A, Medical Laser Applikation (2001); 16; Heft 3: 205-213). Hier lässt sich außer einer Progredienz ein Stillstand oder Rückgang der Läsion kaum nachweisen, da chromogene Substanzen aus eingelagerten Bakterien oder Nahrungsbestandteilen in der Läsion verbleiben würden. Andererseits werden solche Farbstoffe z. B. im Zuge von Bleaching-Behandlungen entfernt, so dass überhaupt keine Aussage mehr möglich ist.

Der Nachteil dieser Methoden liegt darin, dass sie nur Momentaufnahmen der jeweiligen Läsion erlauben und keine Aussagen zum aktuellen Status der Kariesaktivität ermöglichen. Erst bei erneuter Untersuchung sind durch Kariesmonitoring zumindest bei der QLF Aussagen zum Verlauf des kariösen Prozesses - Progression oder Regression - möglich. Auch ist eine Diagnostik erst ab dem Stadium einer subfiziellen Läsion mit Mineralverlust und - bei LF - ab späterer Einlagerung von chromogenen Substanzen und Bakterien möglich. Aufgabe der Erfindung ist es deshalb, die kariöse Läsionsaktivität von Zähnen durch einen einmaligen Messvorgang zu bestimmen.

Gemäß der Erfindung wird die Aufgabe durch eine Messvorrichtung zur Bestimmung des Aktivitätsstatus initialkariöser Schmelzläsionen von Zähnen enthaltend ein zur Vermessung von Poren im Schmelz ausgebildetes optisches Messsystem, das einen in einem Handstück untergebrachten Messkopf mit einer zur Porositäts- und Rauigkeitsmessung ausgebildeten kompakten Sensoroptik aufweist und einen Abstandshalter zur Auflage auf einen Aufsetzpunkt auf der Zahnoberfläche, wobei der Abstandshalter zwischen einer Strahlaus- und -eintrittsfläche des Messkopfes und der Zahnoberfläche einen vorgegebenen festen Messabstand sowie eine Verkippbarkeit des Messkopfes um den Aufsetzpunkt gewährleistet.

Der Messkopf ist in einer bevorzugten Ausgestaltungsvariante der Erfindung über ein optisches Kabel mit einer Lichtquelle und einer Auswerte- und Anzeigeeinrichtung verbunden, wobei das Handstück aus einem Griffelement und einem Kopfteil mit dem Abstandshalter zur Auflage auf der Zahnoberfläche besteht, der zwischen einer Strahlaus- und -eintrittsfläche des Messkopfes und der Zahnoberfläche einen vorgegebenen festen Messabstand gewährleistet. Dabei kann der Messkopf zweckmäßig eine Sensorhülle aufweisen, in die der Abstandshalter integriert ist.

Bei einer anderen Ausgestaltung der Erfindung kann der Messkopf mit einem Strahlscanner ausgestattet sein, der den Messstrahl in einem vorgegebenen Messbereich über die Zahnoberfläche führt.

Das Abstandsmesssystem enthält vorteilhaft eine in dem Messkopf untergebrachte kompakte, geradsichtige Sensoroptik.

Als Mikrooptik kann entweder eine chromatisch-konfokale Sensoroptik oder eine andere konfokale Sensoroptik vorgesehen sein.

Bei chromatisch-konfokal ausgebildeter Sensoroptik steht diese vorzugsweise über einen Strahlteiler mit der Lichtquelle und einem Spektrometer optisch in Verbindung und das optische Messsystem enthält ferner eine Steuer-, Auswerte- und Versorgungseinrichtung, die mit einer Anzeigeeinrichtung, der Lichtquelle und dem Spektrometer verbunden ist. Bevorzugt kann die Sensoroptik über den Strahlteiler neben der Lichtquelle auch mit einem die Intensität messenden optischen Leistungsmessgerät zum Nachweis von rückreflektiertem Licht optisch in Verbindung stehen.

Weitere zweckmäßige und vorteilhafte Ausgestaltungen und Weiterbildungen der erfindungsgemäßen Messvorrichtung ergeben sich aus den abhängigen Ansprüchen.

Das gilt auch für den weiteren Gegenstand der Erfindung, ein Verfahren zur Bestimmung der Oberflächenzustände von Zähnen, bei dem von einer Porenstruktur im Schmelz durch optische Vermessung ein Tiefenprofil erstellt und eine Intensitätsmessung reflektierten Lichtes durchgeführt wird von mindestens einer auf die Schmelzoberfläche scharf fokussierten Wellenlänge, bei der die Reflexionsintensität erfasst wird, wodurch eine durch das Tiefenprofil charakterisierte Porosität oder Rauigkeit der Schmelzoberfläche ermittelt wird.

Bevorzugt erfolgt die optische Vermessung zur Ermittlung der Porosität und Rauigkeit durch eine Kombination eines chromatisch-konfokalen Abstandssensors mit einem Abstandshalter und zusätzlicher Intensitätsmessung, bei der die Abstandsmessung in eine lotrechte Messung der Reflexionsintensität überführt wird, wodurch bei geringer Reflexionsintensität eine hohe Porosität und bei hoher Reflexionsintensität eine geringe Porosität zugeordnet werden kann. Auf Grund der mit unterschiedlicher Porosität und Rauigkeit korrespondierenden Absorptions-und Streulichtverluste des eingestrahlten Abstands-Messlichtes ist es vorteilhaft, wenn bei Kombination eines chromatisch-konfokalen Abstandssensors mit einem Abstandshalter eine alleinige Intensitätsmessung einer auf die Oberfläche fokussierten Wellenlänge erfolgt, deren Reflexionsintensität als Maß für unterschiedliche Rauigkeit bestimmt wird, wobei bei geringer Reflexionsintensität eine hohe und bei hoher Reflexionsintensität eine geringe initial-kariöse Aktivität nachweisbar ist.

Oberflächeninformationen werden somit nicht durch Streuung und Reflexion an tieferen Schmelzschichten beeinflusst.

Eine genaue örtliche Zuordnung der gemessenen Intensität ist gewährleistet.

Intensitätsmessungen können aber gemäß der Erfindung auch vorgesehen sein, ohne dass Abstände von einer Bezugsebene zur Schmelzoberfläche bestimmt werden.

Die auf einer optischen Vermessung der Porentiefe bzw. der Rauigkeit beruhende Erfindung basiert darauf, dass die Kariesaktivität mit der Porosität der Oberflächenzone einer Läsion, der Löslichkeit des verbliebenen Zahnmaterials und der Läsionsumgebung, wie auch mit der Anzahl und Art der Bakterien sowie der Zuckeraufnahme in die Läsion verknüpft ist.

Folglich steigt die Aktivität mit zunehmender Porosität aufgrund einer fortschreitenden Demineralisierung der Läsionsoberfläche. Andererseits kann eine Remineralisierung mit einem superfiziellen Porenverschluss verbunden sein.

Mit der Erfindung gelingt es, durch Ausnutzung quantitativer Informationen zur Porosität und Rauigkeit der Läsionsoberfläche die kariöse Aktivität einer Schmelzläsion bereits durch eine einmalige Vermessung des Tiefenprofils einer im Schmelz ausgebildeten Porenstruktur zu ermitteln.

Die Erfindung soll nachstehend anhand der schematischen Zeichnung näher erläutert werden. Es zeigen:
- Fig. 1: eine Prinzipskizze einer ersten Ausführung der erfindungsgemäßen Messvorrichtung,
- Fig. 2: ein Höhenprofil für einen gesunden Schmelz,
- Fig. 3: ein Höhenprofil für inaktive Schmelzkaries,
- Fig. 4: ein Höhenprofil für aktive Schmelzkaries,
- Fig. 5: eine Prinzipskizze zur chromatisch-konfokalen Abstandsmessung,
- Fig. 6: eine zweite Ausführung einer erfindungsgemäßen Messvorrichtung
- Fig. 7: die Ausführung der Messvorrichtung gemäß Fig. 5 mit Sensoren verschiedener Austrittswinkel des Messlichtes
- Fig. 8: die Abtastung einer Schmelzoberfläche zur Ermittlung der Oberflächenporosität und -rauigkeit
- Fig. 9: den Intensitäts-Peak der scharf fokussierten Wellenlänge
- Fig. 10: das Verhältnis unterschiedlicher Lichtanteile bei der Beleuchtung einer zu untersuchenden Oberfläche
- Fig. 11: das Ergebnis von Intensitätsrriessungen an einer Schmelzfläche mit Attritionsfacette und einem geätzten Bereich (Längenangaben auf x- und γ-Achse in µm) mit daneben angezeigten zugehörigen Intensitätswerten reflektierter Strahlung a ... chromatisch-konfokale Intensitätsmessung b ... konfokale Intensitätsmessung
- Fig. 12: die chromatisch-konfokale Detektion eines Oberflächenprofils
- Fig. 12a: die Handhabung eines Messkopfes bei Intensitätsmessungen zum Auffinden des Maximums der Reflexionsintensität am Ort einer Punktmessung am Beispiel eines geradsichtigen Messkopfes
- Fig. 13: unterschiedliche Ausrichtungen einer konfokalen Sensoroptik zur einer zu untersuchenden Oberfläche
- Fig. 14: minimierte Ausführung des Messsystems mit Siliziumdetektor als Leistungsmesser, Multimodefasern als optische Transmitter und chromatisch-konfokaler Sensoroptik inklusive Abstandshalter.

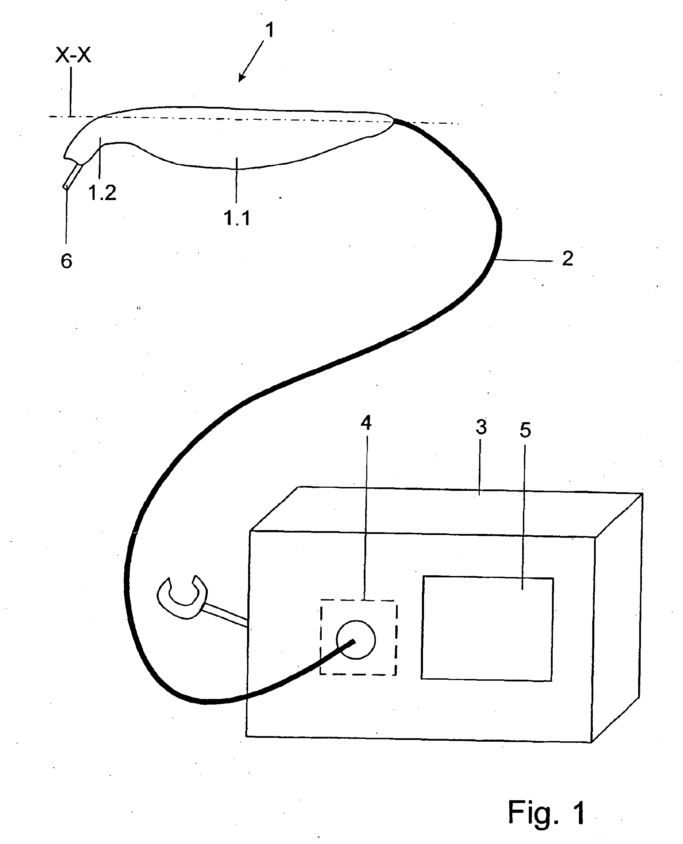

Die erfindungsgemäße Messvorrichtung gemäß Fig. 1 besteht aus einem Handstück 1, in dem ein Messkopf in Form eines Objektives untergebracht ist. Aus dem Handstück 1 ist ein mit dem Messkopf in optischer Verbindung stehendes optisches Kabel 2 mit einer Länge von z. B. 1,5 bis 2 m heraus- und in ein Gehäuse 3 hineingeführt. Das optische Kabel 2 ist in dem Gehäuse 3 mit einer Lichtquelle 4 zur Erzeugung eines Messlichtes und einer Auswerte- und Anzeigeeinrichtung 5, in der das von der Zahnoberfläche reflektierte Messlicht hinsichtlich der optischen Vermessung einer Porenstruktur im Schmelz ausgewertet wird, verbunden.

Das auf die zu untersuchende Zahnoberfläche eines Patienten aufzusetzende Handstück 1, das aus einem Griffelement 1.1 und einem Kopfteil 1.2 besteht, ist einem geknickten optischen Strahlengang angepasst, wofür in dem Messkopf ein geeignetes Strahlumlenkelement vorgesehen ist. Der Bereich, in dem das Kopfteil 1.2 zur Längsachse X-X des Griffelementes 1.1 für den Aus- oder Eintritt des Messlichtes abgewinkelt ist, sollte bevorzugt zwischen 90 und 135 Grad liegen.

Das Kopfteil 1.2 ist mit einem benachbart zur Strahlaus- und -eintrittsfläche des Messkopfes liegenden Abstandshalter 6 ausgestattet, der den notwendigen definierten Messabstand zur Zahnoberfläche gewährleistet.

Eine Messung von Höhenunterschieden in einem zum Aufsetzpunkt des Abstandshalters 6 benachbarten Bereich der Zahnoberfläche kann entweder durch Verkippung des Kopfteils 1.2 um ca. +/- 20 Grad gegen die Senkrechte auf der Zahnoberfläche erfolgen oder der Messkopf wird mit einem Strahlscanner, wie z. B. einem Schwingspiegel, ausgestattet, der den Messstrahl durch Strahlablenkung in einem vorgegebenen Messbereich über die Zahnoberfläche führt.

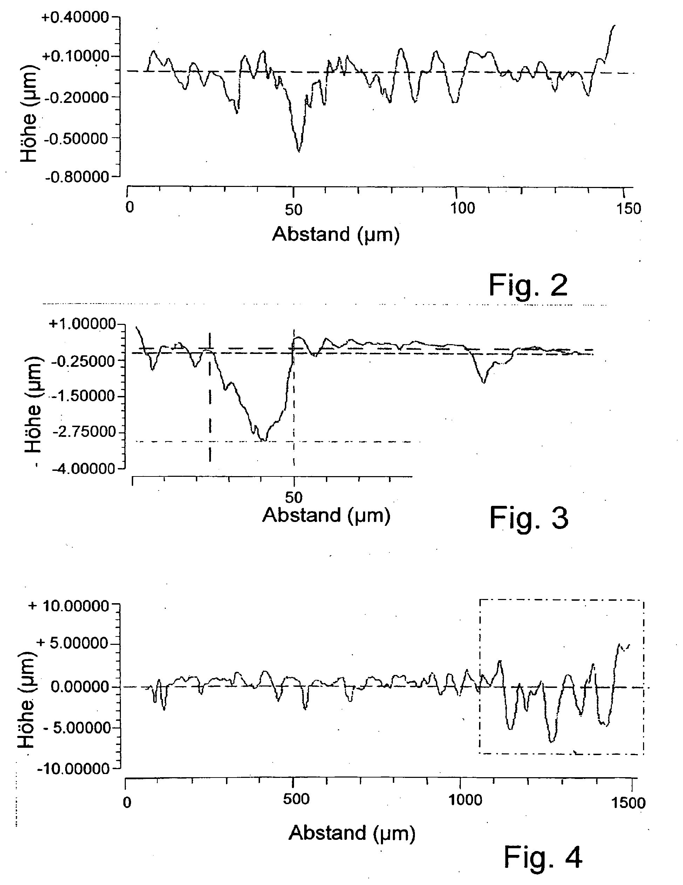

Mit der erfindungsgemäßen Vorrichtung lassen sich anhand gemessener Höhenprofile, wie sie in den Figuren 2 bis 4 dargestellt sind, gesunde Schmelzbereiche von aktiven und inaktiven Läsionsbereichen unterscheiden. Während gesunde Schmelzbereiche nur geringe Höhendifferenzen z. B. um 0,5 µm aufweisen, liegen diese bei aktiven Läsionsbereichen z. B. um 10 µm und bei inaktiven Läsionsbereichen z. B. um 5 µm.

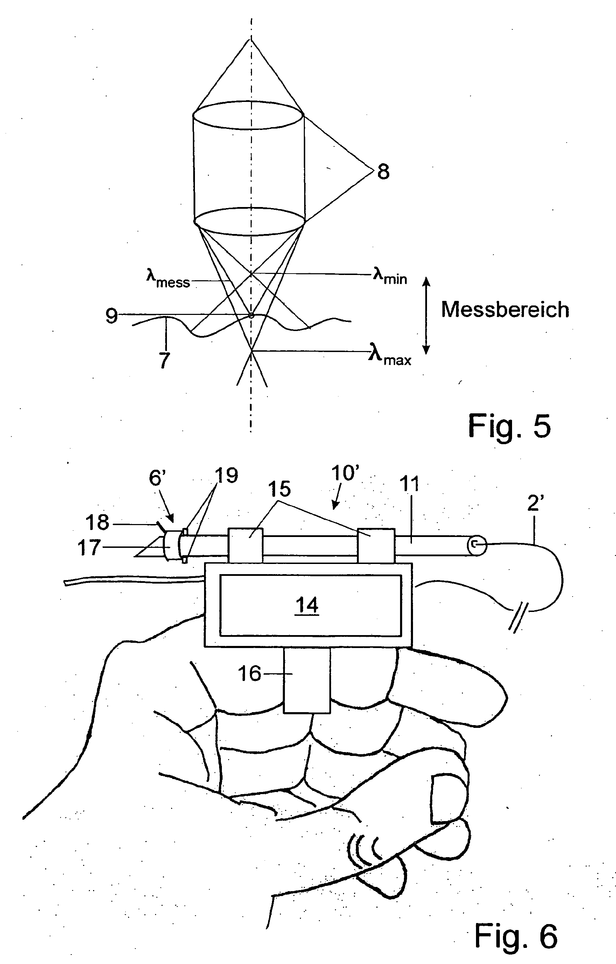

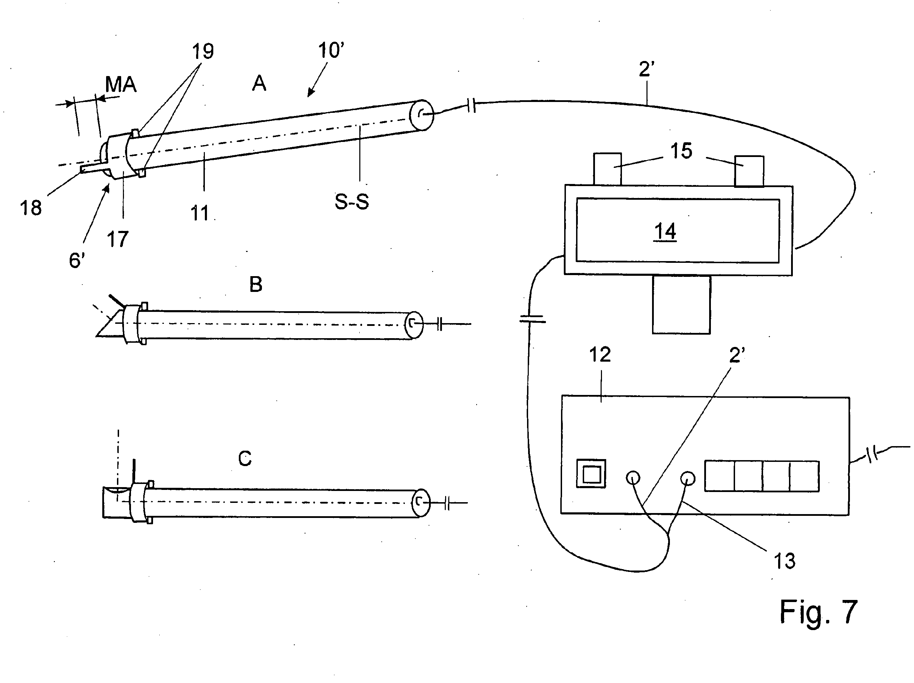

Sowohl für die Ausführung der Messvorrichtung gemäß Fig. 1 als auch für eine zweite Ausführung gemäß Fig. 6 und 7 wird das bekannte Verfahren einer chromatisch-konfokalen Detektion in Form einer Abstandsmessung bevorzugt, bei welcher der chromatische Längsfehler einer speziellen Optik genutzt wird. Polychromatisches Licht wird von einem Bezugspunkt; der einen definierten Abstand zu der zu messenden Oberfläche 7 aufweist, über eine Sensoroptik 8 mit ausgeprägtem Farblängsfehler wellenlängenabhängig auf diese Oberfläche 7 fokussiert. Es entsteht ein Messpunkt 9 mit sehr geringem Durchmesser, für den sich jedoch nur für eine Wellenlänge λₘₑₛₛ eine scharfe Abbildung auf der Oberfläche 7 ergibt (Fig. 5).

Das reflektierte Licht dieser Wellenlänge λₘₑₛₛ wird auf dem Rückweg durch die Sensoroptik gleichfalls scharf auf die Eintrittsöffnung des optischen Kabels 2' abgebildet. Alle anderen Wellenlängen sind wegen der unscharfen Abbildung unwirksam. Hinter dem optischen Kabel 2' gelangt das Licht in nicht dargestellter Weise über einen Strahlenteiler oder eine Faserweiche in ein Spektrometer, in dem die betreffende Wellenlänge λₘₑₛₛ ein scharfes Intensitätsmaximum zeigt. Entsprechend vorgesehener Kalibrierung kann aus der ermittelten Wellenlänge λₘₑₛₛ der zugehörige Abstand im Nanometerbereich zwischen dem Bezugspunkt der Sensoroptik und der zu untersuchenden Oberfläche 7 bestimmt werden.

Bei einer zweiten Ausführung der erfindungsgemäßen Messvorrichtung (Fig. 6 und 7) ist ein Messkopf 10' vorgesehen, der eine Sensoroptik aus einer kompakten, passiven Mikrooptik ohne elektronische oder bewegte Bauteile enthält, die einen Durchmesser von 1 mm bis 2 mm besitzt und von einer zylindrischen Sensorhülle 11 umgeben ist.

Die Sensoroptik erzeugt einen Meßfleckdurchmesser zwischen 2 µm und 5 µm und weist eine laterale Auflösung zwischen 1 µm und 2,5 µm und eine vertikale Auflösung zwischen 10 nm und 100 nm auf. Für die Untersuchung einer Zahnoberfläche ist ein Meßabstand zwischen 4 mm und 10 mm geeignet.

Der Messbereich für die Abstandsmessung liegt innerhalb des Brennweitenbereichs des Farblängsfehlers, wobei für die Messung an Zahnoberflächen ein Arbeitsbereich zwischen 300 µm und 6500 µm sinnvoll ist.

Eine Steuer-, Auswerte- und Versorgungseinrichtung 12 enthält eine Lichtquelle, einen Strahlenteiler, ein Spektrometer und eine Steuer- und Auswerteelektronik. Die Steuer-, Auswerte- und Versorgungseinrichtung 12 kann als Standgerät oder als PC-Karte ausgeführt sein. Sie kann auch in zahnärztliche Behandlungsstühle integriert sein.

Gemeinsam mit einem optischen Kabel 2' wird aus der Steuer-, Auswerte- und Versorgungseinrichtung 12 ein Steuerkabel 13 herausgeführt, das den Betrieb einer Anzeigeeinrichtung 14 ermöglicht. Die Anzeigeeinrichtung 14 besitzt eine Halterung 15 für die Befestigung des Messkopfes 10' und kann über einen Clip oder Ring 16 an einem Finger der freien Hand getragen werden.

Sofern im Zuge einer zukünftigen weiteren Komponentenminiaturisierung die örtliche Trennung zwischen der Steuer-, Auswerte- und Versorgungseinrichtung 12 und der Sensoroptik durch ein langes optisches Kabel (Lichtleitkabel) nicht notwendig ist, können beide Elemente auch in ein graziles Handgerät integriert sein, welches direkt im Mund des Patienten angewendet werden kann.

Alternativ kann eine komplett miniaturisierte Steuer-, Auswerte- und Versorgungseinrichtung mit Anzeige an einem Fingerclip getragen werden und durch ein Lichtleitkabel mit einer Mikrooptik als Sensoroptik verbunden sein. Die Stromversorgung erfolgt dann jeweils über Akku- oder Batteriebetrieb.

Da sich mit dieser Meßvorrichtung Abstände bzw. Höhendifferenzen zu einem Bezugspunkt mit einer vertikalen Auflösung (z-Achse) um 10 nm messen lassen, besteht eine ausreichende Sensitivität für die nachzuweisenden Höhenunterschiede wischen 0,5 µm und 10 µm.

Ebenso wie die Ausführung gemäß Fig. 1 weist auch die zweite Ausführung der erfindungsgemäßen Messvorrichtung einen Abstandshalter 6' auf, um bei der Untersuchung von Zahnoberflächen einen Bezugspunkt realisieren zu können und die Einhaltung des Meßabstandes MA der Sensoroptik zu sichern.

Der Abstandshalter 6', der aus einem Abstandshalterring 17 und einem dem Winkel des Lichtaustritts entsprechenden Abstandshalterarm 18 besteht, ist auf die Sensorhülle 11 bis an Abstandshalternasen 19 an der Sensorhülle 11 herangeschoben. Die im sensorseitigen Ende des Messbereiches liegende Stirnfläche des Abstandshalterarms 18 ist aufgeraut, um ein Verrutschen auf der Zahnoberfläche zu verhindern.

Der Abstandshalter 6' ist gemäß Fig. 7 unterschiedlichen Abstrahlwinkeln des Messkopfes 10' angepasst, in dem der Abstandshalterarm 18 zur Achse S-S der Sensorhülle derart unterschiedlich geneigt ist, dass er in die jeweilige Abstrahlrichtung weist. Die wegen der Übersichtlichkeit ausschließlich mit Bezugszeichen versehene Ausführung A entspricht einer geradsichtigen Sensoroptik, B einer um 135° und C einer um 90° gegenüber der Achse S-S der Sensorhülle 11 geneigten Abstrahlrichtung. Mit den Varianten B und C lassen sich auch schwer zugängliche Seitenzahnflächen erreichen. Für den gewinkelten Strahlengang ist innerhalb der Sensorhülle 11 ein geeignetes Prisma vorgesehen.

Alternativ lässt sich der Abstandshalter 6' auch direkt in die Sensorhülle 11 integrieren. Eine weitere Alternative besteht darin, die Mikrooptik so zu berechnen und zu gestalten, dass der Messbereich unmittelbar an der Lichtaustrittsfläche der Mikrooptik (für die geradsichtige Sensoroptik) oder des Prismas (für den Winkelsensor) beginnt. Die Funktion des Abstandshalters ist dann in die Sensoroptik integriert und ein separater Abstandshalter kann entfallen. Die Sensoroptik kann dann direkt auf die zu untersuchende Oberfläche aufgesetzt werden. Für noch zu beschreibende Intensitätsmessungen muss jedoch verhindert werden, dass Streulicht an der Innenwand der direkt aufsitzenden Sensorhülle 11 in die Sensoroptik reflektiert wird, denn dadurch würde ein höherer Intensitätswert vorgetäuscht werden.

Zur Benutzung am Patienten wird der Messkopf 10' mit einem Einwegüberzug aus dehnbarer Folie überzogen. Der Abstandshalter 6' ist entweder sterilisierbar oder als Einwegartikel ausgeführt.

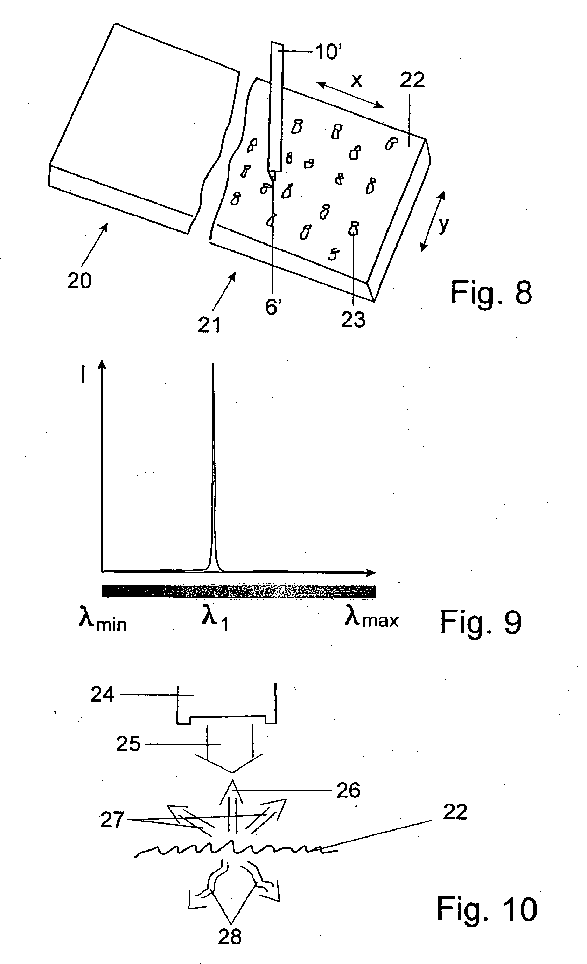

Die erfindungsgemäße Messvorrichtung gestattet gemäß Fig. 8 Porositätsmessungen an inaktiven und aktiven Läsionsarealen 20, 21, die in folgender Weise durchgeführt werden können.

Das zu untersuchende Zahnareal wird zunächst mittels 5%-iger Natriumhypochloridlösung von Plaque gesäubert. Die Applikation erfolgt mittels eines zahnärztlichen Adhäsiv-Tip's.

Durch Seitwärtsbewegungen des mit dem Abstandshalter 6' auf die Schmelzoberfläche 22 aufgesetzten Messkopfes 6' in x- und γ-Richtung können durch Poren 23 in der Schmelzoberfläche 22 hervorgerufene Abstandsunterschiede zum Bezugspunkt der Sensoroptik ermittelt und an der Anzeige 14 ausgegeben werden.

Das Vorhandensein oder die Abwesenheit von Abstandsunterschieden erlaubt eine Aussage zur Oberflächenporosität bzw. -rauigkeit und damit zur Läsionsaktivität. Sind hinreichende Abstandsunterschiede vorhanden, dann liegt Porosität und Rauigkeit und damit Läsionsaktivität vor. Sind keine Abstandsunterschiede vorhanden, ist die Läsionsoberfläche geschlossen und die Läsion ist inaktiv.

Zur Differenzierung der Läsionsaktivität lässt sich zusätzlich zum Abstandsunterschied auch der Intensitätswert der "Abstandswellenlänge" auswerten.

Das Messprinzip der beschriebenen Abstandsmessung ist unabhängig von den Oberflächeneigenschaften einer Messprobe (Reflektivität, Farbe und Textur). Zur Abstandsmessung wird allein die Lage des Intensitäts-Peaks der scharf fokussierten Wellenlänge auf der Wellenlängen-Achse (x-Achse) entsprechend Fig. 9 ausgewertet.

Die unterschiedliche Höhe dieses Intensitäts-Peaks (y-Achse) ermöglicht bei Oberflächen mit unterschiedlichen Oberflächenzuständen zusätzlich eine Differenzierung der Rauigkeit am Messort, wodurch sich unterschiedliche Oberflächenzustände bzw. Rautiefen desselben Materials unterscheiden lassen.

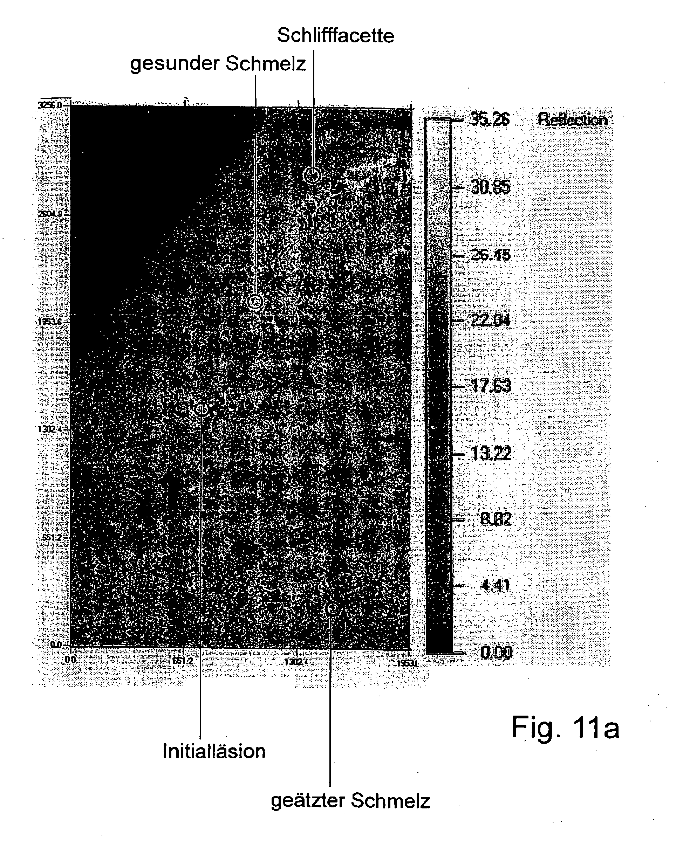

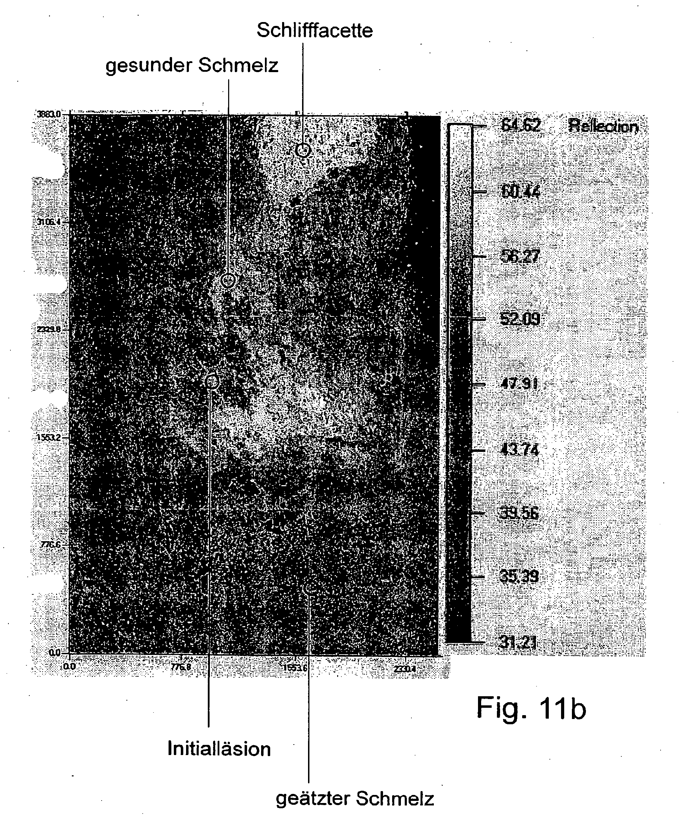

Untersucht man vergleichsweise im Labor mittels eines stationären chromatisch-konfokalen Abstandsmesssystems mit einem Flächenscan Zahnoberflächen mit unterschiedlichen Aktivitätszuständen (z. B. gesunden und geätzten Schmelz, aktive und inaktive Initialläsionen oder auch Erosionen), treten bei geringen Rauigkeiten bzw. glatten Oberflächen hohe Intensitätswerte und bei rauen Oberflächen geringe Intensitätswerte auf (Fig. 11a; b).

Der Intensitätswert der reflektierten Wellenlänge (spiegelnde Reflektion) ist ein Maß für die nicht durch Streuung (streuende Reflektion) und Absorption verloren gegangenen Anteile des lotrecht auf die Schmelzoberfläche 22 eingestrahlten Messlichtes. Fig. 10 verdeutlicht das Verhältnis der Lichtanteile bei der Reflexion. Es bedeuten:
24 - Sensoroptik
25 - eingestrahlte Lichtmenge
26 - reflektierter Lichtanteil für Abstands- und Intensitätsmessungen
27 - gestreute Lichtanteile
28 - absorbierte Lichtanteile

Das Verhältnis von spiegelnder Reflektion zu streuender Reflektion und Absorption hängt dabei von der Rautiefe der Schmelzoberfläche 22 ab (Lexikon der Optik, Teil 2, S. 251, Spektrum Akademischer Verlag Heidelberg, Berlin 2003). Danach erfolgt spiegelnde Reflexion, wenn die Rautiefe klein gegenüber der Wellenlänge ist, während streuende Reflexion bei einer Rautiefe in der Größenordnung der Wellenlänge auftritt. Über Streulichtmessungen am selben Messort lässt sich eine Beziehung zu Rauigkeitsparametern (z. B. root-mean-square-(rms)-Rauheit) herstellen. Das Verhältnis Reflexions- zu Streulichtintensität und damit zu rms-Werten kann dann über Eichmessungen hergestellt werden.

Die Reflexionsintensität ist allgemein abhängig von der Messrate, der Neigung der zu vermessenden Oberfläche zum Messkopf, des Reflexionsvermögens der Oberfläche für die Detektionswellenlänge, der Helligkeit der Lichtquelle, welche die Wellenlänge bereitstellt und der Reaktion des Spektrometers bei dieser Wellenlänge.

Der zu ermittelnde Intensitätswert wird üblicherweise als Integral der durch die jeweilige Detektionswellenlänge überdeckten Fläche berechnet. Trotz gering unterschiedlicher Peakmaxima der verschiedenen Wellenlängen ergibt die Auswertung wegen gleicher Flächen gleiche Intensitätswerte für gleiche Rautiefen.

Unabhängig davon ist der wellenlängenbedingte Unterschied der Peakmaxima klein im Verhältnis zu den am Zahnschmelz untersuchten Intensitätsunterschieden und somit vernachlässigbar.

Die für die Intensitätsmessungen vorgesehene Messrate ist bevorzugt so einzustellen, dass unterhalb der Sättigungs Intensitätswerte zwischen 0 und ca. 50% gemessen werden.

Bei einer alleinigen Punktmessung wird bereits eine Aussage über den Oberflächenzustand am Messort möglich. Für eine solche Punktmessung ist zur Steigerung der Aussagesicherheit eine Vergrößerung des Messfleckdurchmessers des Sensors auf 10 µm sinnvoll. Dadurch verringert sich die vertikale Auflösung zwar auf 100 nm, wodurch die Intensitätsmessung jedoch nicht beeinflusst wird.

Vorteilhaft können an der Steuer-, Auswerte- und Versorgungseinrichtung 12 im Wechsel mehrere Messköpfe 10' betrieben werden. Alternativ können aber auch Steuer-, Auswerte- und Versorgungseinrichtungen mit mehreren Steckplätzen zur Anwendung kommen.

Da die bei der Erfindung vorgesehene Intensitätsmessung nur für scharf auf die Schmelzoberfläche 22 fokussierte Wellenlängen erfolgt, enthält die ermittelte Intensität im Vergleich zu anderen Reflexionsmessanordnungen (z. B. kamerabasiert) ausschließlich Oberflächeninformationen und wird nicht durch Streuung und Reflexion aus tieferen Schmelzschichten beeinflusst.

Die zur unterstützenden . Intensitätsmessung vorgesehene Sensoroptik ist in einer Ausgestaltung der Erfindung, wie bereits beschrieben, als chromatisch-konfokale Sensoroptik ausgebildet.

Zur Messung der Intensität der Reflexion wird die Sensoroptik mit dem Abstandshalter 6' auf das interessierende Areal der Schmelzoberfläche 22 aufgesetzt. An der Anzeigeeinrichtung 14 wird die für diesen Ort gemessene Intensität als Zahlenwert abgelesen.

Zur Intensitätsmessung kann alternativ eine ebenfalls für Abstandsmessungen geeignete rein konfokale Sensoroptik vorgesehen sein. Bei einer zur chromatisch-konfokalen Sensoroptik vergleichsweise ähnlich guten Differenzierung der Oberflächenzustände (Fig. 11 b) ist ein geringerer Geräteaufwand zur Auswertung notwendig.

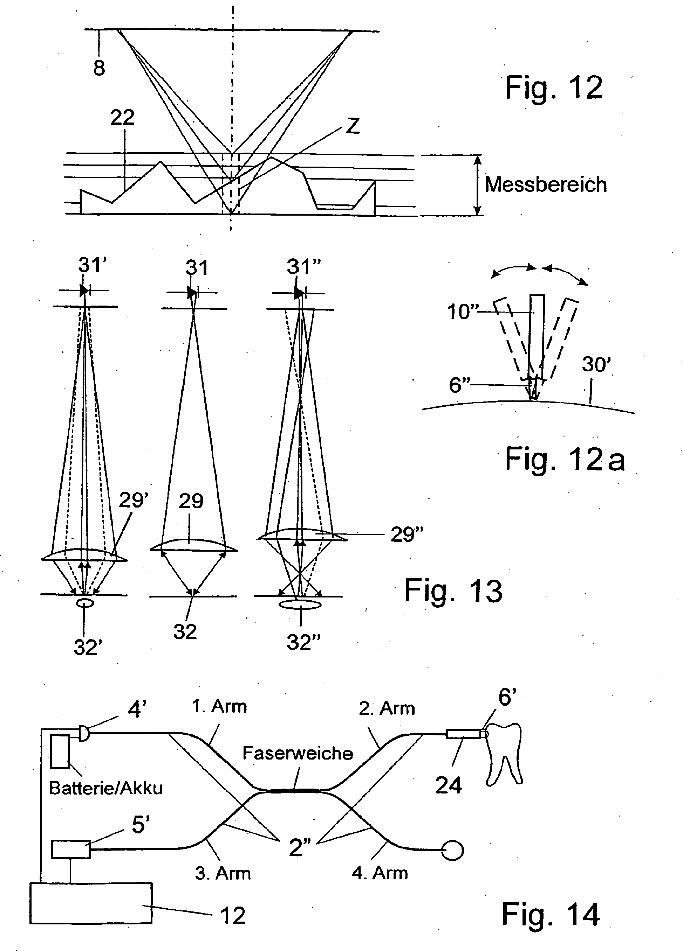

Bei chromatisch-konfokaler Sensoroptik wird der Intensitätswert wegen des chromatischen Tiefenscans an demjenigen Profilanteil der Oberfläche generiert, der innerhalb des gedachten Zylinders Z aus Messfleckdurchmesser und Länge des Messbereiches liegt (Fig. 12). Solange die Schmelzoberfläche 22 in diesem Meßbereich liegt, wird für die jeweils scharf auf diesen Oberflächenbereich fokussierte Wellenlänge ein Intensitätswert ermittelt.

Im Gegensatz dazu wird bei der rein konfokalen Sensoroptik eine punktförmige Lichtquelle mit einem Abtastobjektiv 29 ohne Farblängsfehler auf die Oberfläche 30 einer Probe abgebildet (Fig. 13). Das reflektierte Licht wird vom selben Objektiv 29 eingesammelt und auf einen punktförmigen Detektor 31 geleitet.

Eine geeignete Einrichtung zur konfokalen Abstands- und Intensitätsmessung enthält in nicht dargestellter Weise eine Lichtquelle für weißes oder monochromatisches Licht, eine Faserweiche zur Einkopplung des zur konfokalen Sensoroptik führenden Lichtes und zur Auskopplung des rückreflektierten Lichtes. Dessen Intensität wird mit einem optischen Leistungsmessgerät ausgewertet und als Zahlenwert ausgegeben.

Bei Abstandsmessungen führt das Sensorobjektiv üblicherweise harmonische Schwingungen aus, da eine Fokussierung nur in einer Ebene möglich ist.

Bei im Fokus befindlicher Oberfläche ist die Leuchtdichte maximal (mittlere Darstellung in Fig. 13) und fällt mit zunehmender Defokussierung (rechte und linke Darstellung in Fig. 13) rasch ab. Bei der Abbildung zurück wird die beleuchtete Fläche 32 auf den Detektor 31 abgebildet. Bei Defokussierung wird die Strahlung auf eine große Fläche 32', 32" verteilt, die Leuchtdichte auf dem Detektor 31', 31" ist gering. Die Abbildung weist einen quadratischen Intensitätsabfall auf. Insgesamt ergibt sich ein scharf ausgeprägtes Intensitätsmaximum auf dem Detektor 31, sobald sich die Oberfläche 30 im Fokus befindet. Eine nicht dargestellte Auswerteelektronik erfasst den Zeitpunkt des Signalmaximums und errechnet den zugehörigen Profilwert.

Zur Intensitätsmessung entsteht im Vergleich zu einem Messkopf mit chromatisch-konfokaler Sensoroptik mit Abstandhalter bei gleicher Handhabung eines Messkopfes mit konfokaler Sensoroptik mit Abstandshalter nur eine scharf fokussierte Ebene. Diese muss in einem Bereich innerhalb der höchsten und niedrigsten Erhebung der zu untersuchenden Oberfläche liegen.

Bei ungünstiger Oberflächenwölbung kann die Fokusebene mitunter außerhalb dieses Bereiches zu liegen kommen. Dann kann alternativ eine chromatisch-konfokale Sensoroptik genutzt werden, deren Meßbereich die Oberflächenwölbung berücksichtigt. Die Reflexionsintensität kann mit einem optischen Leistungsmeßgerät gemessen werden.

Für die hier durchgeführte Reflexionsmessung wird ein gezielt ausgewählter enger Wellenlängenbereich innerhalb des Messbereichs genutzt, um die unterschiedliche Reflexionsintensität desselben Wellenlängenbereichs auf unterschiedlichen Oberflächenzuständen desselben Materials (Zahnschmelz oder Wurzeldentin) zu bestimmen. Die Auswahl des Wellenlängenbereichs wird durch den Abstandshalter erreicht. Durch die Kombination des chromatisch-konfokalen Sensors mit einem Abstandshalter erfolgt eine Umstellung der Abstandsmessung auf eine Messung der lotrechten Reflexionsintensität. Da die Reflexionsintensität auch in geringem Maße von der Wellenlänge abhängt, wird derjenige Wellenlängenbereich benutzt, für den der Zahnschmelz die höchste Reflexion (blau-grün) und die geringste Absorption besitzt. Zum roten Wellenlängenbereich hin sind die Reflexionswerte etwas geringer, da rotes Licht in tiefere Probenschichten eindringt.

Die Größe des notwendig zu nutzenden Wellenlängenbereichs ergibt sich aus dem Peak-Valley-Maximum der Probenfläche (hier 10 µm) aus einem Messbereich - je nach benutztem Sensor - von 300 bis 6500 µm. Durch die Einengung des Wellenlängenbereichs werden Messfehler aufgrund der Wellenlängenabhängigkeit der Reflexionsintensität reduziert. Durch perfekte Fokussierung des chromatischen Messsystems wird nur reflektiertes Licht der Oberflächenschicht detektiert. Die manuelle Positionierung lässt selektive Punktmessungen an Zahnoberflächen zur Differenzierung der Läsionsaktivität zu.

Soll ausschließlich die Reflektionsintensität bestimmt werden, kann die gerätetechnische Anordnung dahingehend vereinfacht werden, dass anstelle des Spektrometers nur mit einem optischen Leistungsmesser, bestehend aus einem Siliziumdetektor, Auswerteelektronik und Intensitätsanzeige ausgewertet wird.

Das so vereinfachte Messsystem kann weitergehend miniaturisiert werden und besteht dann - wie in Fig. 14 dargestellt - aus einer batterie- oder akkubetriebenen Weißlicht-LED 4', die die netzbetriebene Halogenlichtquelle 4 ersetzt, wobei mittels Multimodefasern 2" über eine Faserweiche 2"' als Strahlteiler die Weißlicht-LED 4' sowohl mit der chromatisch-konfokalen Sensoroptik 24 als auch mit einem Siliziumdetektor 5' (als vereinfachtes Leistungsmess- und Anzeigemodul 5) in Verbindung steht. Die Faserweiche 2"' weist dabei vier Faserarme auf, von denen der erste Arm an die Weißlicht-LED 4' angekoppelt ist, der zweite Arm der chromatisch-konfokalen Sensoroptik 24, die mit einem Abstandshalter 6' auf die Schmelzoberfläche 22 eines Zahnes aufgesetzt ist, zugeführt wird und der dritte Arm mit dem Siliziumdetektor 5' zur Reflexionsintensitätsmessung und Intensitätsanzeige verbunden ist. Der vierte Arm ist ungenutzt auf eine Lichtfalle gelegt.

Die Baugröße eines Messkopfes 10" mit einer konfokalen Sensoroptik entspricht der des Messkopfes 10' mit chromatisch-konfokaler Sensoroptik.

Zur Ermittlung der Reflexionsintensitätswerte wird der Messkopf 10" mit dem Abstandhalter 6" lotrecht auf die zu untersuchende Oberfläche 30' aufgesetzt. Die lotrechte Sensorposition auf die Tangente der Oberfläche 30' wird ermittelt, indem durch geringfügige Verkippung des Messkopfes 10" in Pfeilrichtung das Maximum der Reflexionsintensität am Ort der Punktmessung gesucht wird (Fig. 12a).

Der Maximalwert der Reflexionsintensität er veränderten Oberfläche lässt sich dann in Relation zum Maximalwert der Reflexionsintensität der nicht veränderten Oberfläche setzen.

Zur Differenzierung des Aktivitätsstatus können die Parameter Abstandsunterschied und Reflexionsintensität bewertet werden. Mit beiden Verfahren, Abstandsmessung und Intensitätsmessung, wird jeweils eine Messung in einem gesunden unveränderten Schmelzareal als Bezugswert durchgeführt. Dann wird das zu bewertende, veränderte Schmelz- bzw. Zahnareal untersucht.

Läsionen, deren Messwerte eher dem des gesunden Bereiches bzw. Referenzbereiches nahe kommen, werden als inaktiv bewertet. Läsionen, deren Messwerte eher der Hälfte des Bezugswertes nahe kommen, werden als aktiv bewertet.

Klassifizierungsbeispiele zur Bestimmung des Aktivitätsstatus initialkariöser Schmelzläsionen können folgende Intensitätswerte im Vergleich zur eingestrahlten Lichtmenge aufweisen:

| | | |
|---|---|---|
| Messrate 300 Hz | - gesund | ca. 50 % |
| | - inaktiv | ca. 40 % |
| | - aktiv | ca. 20 % |
| Messrate 1000 Hz | - gesund | ca. 20%. |
| | - inaktiv | ca. 16 % |
| | - aktiv | ca. 2 %. |

Die erfindungsgemäße Messvorrichtung lässt sich auch für andere Anwendungen verwenden.

Neben der Untersuchung initialkariöser Läsionen nimmt die Untersuchung von erosiven Zahnveränderungen einen breiten Raum im zahnmedizinischen Schrifttum ein. Erosiver Substanzverlust ist dabei multifaktoriell durch säurehaltige Getränke und Nahrungsmittel, exzessive Zahnputztechniken, craniomandibuläre Dysfunktionen mit Überbelastung einzelner Zähne und weitere Ursachen bedingt. Der Substanzverlust betrifft Zahnhälse und Kauflächen. Mittels Intensitätsmessung lassen sich die Ursachen differenzieren. Säurebedingte Erosionen bewirken eher stumpfe, matte Oberflächen mit geringer Reflexionsintensität. Exzessive Putztechniken oder Abrasionen bewirken eher glatte, glänzende Oberflächen.

Die Intensitätsmessung lässt sich auch zur Differenzierung weiterer Oberflächenzustände, wie der Beurteilung der Politurgüte zahnärztlicher Restaurationen oder bei der Untersuchung technischer Oberflächen einsetzen. Es kann somit auch eine quantitative Charakterisierung von Oberflächenveränderungen eines Untersuchungsobjektes im Sinne der Bestimmung einer zustandsabhängigen Materialkenngröße vorgenommen werden.

Eine weitere Alternative kann eine alleinige Intensitätsmessung mit einer rein konfokalen Sensoroptik sein.

## Patentansprüche

1. Messvorrichtung zur Bestimmung des Aktivitätsstatus initialkariöser Schmelzläsionen von Zähnen enthaltend
- ein zur Vermessung von Poren im Schmelz ausgebildetes optisches Messsystem, das einen in einem Handstück (1) untergebrachten Messkopf (10', 10") mit einer Sensoroptik (24), bestehend aus einer kompakten, passiven Mikrooptik zur Porositäts- und Rauigkeitsmessung, aufweist und
- einen Abstandshalter (6, 6', 6") zur Auflage auf einen Aufsetzpunkt auf der Zahnoberfläche, wobei der Abstandshalter (6, 6', 6") zwischen einer Strahlaus- und -eintrittsfläche des Messkopfes (10', 10") und der Zahnoberfläche einen vorgegebenen festen Messabstand sowie eine Verkippbarkeit des Messkopfes (10', 10") um den Aufsetzpunkt gewährleistet.

2. Messvorrichtung nach Anspruch 1, wobei der Messkopf (10', 10") eine Sensorhülle (11) aufweist, in die der Abstandshalter (6, 6', 6") integriert ist.

3. Messvorrichtung nach Anspruch 1 oder 2, wobei der Messkopf über ein optisches Kabel (2) mit einer Lichtquelle (4) und mit einer Auswerte- und Anzeigeeinrichtung (5) verbunden ist und das Handstück (1) aus einem Griffelement (1.1) und einem Kopfteil (1.2) mit dem Abstandshalter (6) besteht.

4. Messvorrichtung nach Anspruch 3, wobei der Messkopf (10', 10") mit einem Strahlscanner ausgestattet ist, der den Messstrahl in einem vorgegebenen Messbereich über die Zahnoberfläche führt.

5. Messvorrichtung nach einem der Ansprüche 1 bis 3, wobei das Messsystem eine in dem Messkopf (10', 10") untergebrachte geradsichtige Sensoroptik (24), enthält.

6. Messvorrichtung nach einem der Ansprüche 1 bis 3, wobei das Messsystem eine in dem Messkopf (10', 10") untergebrachte Sensoroptik (24), die einen gewinkelten Strahlengang in einem Winkelbereich von 90° bis 135° aufweist, enthält.

7. Messvorrichtung nach Anspruch 5 oder 6, wobei die Sensoroptik (24) zur Erzeugung eines Messfleckdurchmessers zwischen 2 µm und 10 µm ausgebildet ist, eine laterale Auflösung zwischen 1 µm und 5 µm, eine vertikale Auflösung zwischen 10 nm und 100 nm und einen Arbeitsbereich innerhalb eines Brennweitenbereichs des Farblängsfehlers der Sensoroptik (24) zwischen 300 µm und 1500 µm aufweist.

8. Messvorrichtung nach Anspruch 5 oder 6, wobei die Mikrooptik als chromatisch-konfokale Sensoroptik (24)
ausgebildet ist und über einen Strahlteiler mit der Lichtquelle und einem Spektrometer optisch in Verbindung steht und das optische Messsystem weiterhin eine Steuer-, Auswerte- und Versorgungseinrichtung (12) enthält, die mit einer Anzeigeeinrichtung (14), der Lichtquelle und dem Spektrometer verbunden ist.

9. Messvorrichtung nach Anspruch 7, wobei die Mikrooptik als konfokale Sensoroptik (24) ausgebildet ist und über einen Strahlteiler mit der Lichtquelle und einem die Intensität messenden optischen Leistungsmessgerät zum Nachweis von rückreflektiertem Licht optisch in Verbindung steht und das optische Messsystem weiterhin eine Steuer-, Auswerte-und Versorgungseinrichtung (12) enthält, die mit einer Anzeigeeinrichtung (14), der Lichtquelle und dem Leistungsmessgerät verbunden ist.

10. Messvorrichtung nach Anspruch 8, wobei mittels der Kombination der chromatisch-konfokalen Sensoroptik mit dem Abstandshalter eine chromatisch-konfokale Abstandsmessung in eine lotrechte Messung der Reflexionsintensität mittels des Spektrometers überführbar ist, wodurch bei geringer Reflexionsintensität eine hohe kariöse Aktivität und bei hoher Reflexionsintensität eine geringe kariöse Aktivität nachweisbar ist.

11. Messvorrichtung nach Anspruch 9, wobei mittels der Kombination einer chromatisch-konfokalen Sensoroptik mit dem Abstandshalter eine chromatisch-konfokale Abstandsmessung in eine lotrechte Messung der Reflexionsintensität mittels des Leistungsmessgerätes überführbar ist, wodurch bei geringer Reflexionsintensität eine hohe kariöse Aktivität und bei hoher Reflexionsintensität eine geringe kariöse Aktivität nachweisbar ist.

12. Messvorrichtung nach Anspruch 10 oder 11, wobei eine Detektion als alleinige Intensitätsmessung reflektierten Lichtes von mindestens einer auf die Oberfläche scharf fokussierten Wellenlänge vorgesehen ist, bei der die Reflexionsintensität erfasst wird, wodurch eine durch das Tiefenprofil charakterisierte Porosität oder Rauigkeit der Schmelzoberfläche ermittelbar ist.

13. Messvorrichtung nach Anspruch 8, wobei der Messkopf (10') mittels eines optischen Kabels (2') über den Strahlteiler an die Lichtquelle und an das Spektrometer angeschlossen ist und die Anzeigeeinrichtung (14) über ein Steuerkabel (13) mit der Steuer-, Auswerte- und Versorgungseinrichtung (12) in Verbindung steht.

14. Messvorrichtung nach Anspruch 13, wobei Mittel zur Befestigung der Anzeigeeinrichtung (14) an einem Finger einer Hand und eine Halterung (15) zur temporären Halterung des Messkopfes (10') an der Anzeigeeinrichtung (14) vorhanden sind.

15. Messvorrichtung nach einem der Ansprüche 8 bis 13, wobei die Lichtquelle, das Spektrometer und die Steuer-, Auswerte- und Versorgungseinrichtung (12) gemeinsam als PC-Karte ausgebildet sind.

16. Messvorrichtung nach einem der Ansprüche 8 bis 13, wobei die Lichtquelle, das Spektrometer und die Steuer-, Auswerte- und Versorgungseinrichtung (12) gemeinsam als Einbaugerät in zahnärztliche Behandlungsstühle ausgebildet sind.

17. Verwendung einer Messvorrichtung gemäß einem der Ansprüche 1 bis 16, zur Differenzierung von Zahnoberflächenzuständen, die durch erosiven Substanzverlust geprägt sind.

18. Verwendung einer Messvorrichtung gemäß einem der Ansprüche 1 bis 16 zur Beurteilung der Politurgüte zahnärztlicher Restaurationen.

19. Verwendung einer Messvorrichtung gemäß einem der Ansprüche 1 bis 16 zur quantitativen Charakterisierung von Oberflächenzuständen technischer Oberflächen im Sinne der Bestimmung einer zustandsabhängigen Materialkenngröße.

## Claims

1. Measuring apparatus for determining the activity status of initial carious enamel lesions of teeth comprising an optical measuring system which is designed for measuring pores in the enamel and which has a measuring head (10', 10") accommodated in a handpiece (1), this measuring head (10', 10") having compact, passive microoptics for measuring porosity and roughness, and a spacer (6, 6', 6") for resting on a placement point on the surface of the tooth, wherein the spacer (6, 6', 6") guarantees a predetermined fixed measuring distance between a beam exit face of the measuring head (10', 10") and a beam entrance face of the measuring head (10', 10") and the tooth surface and ensures that the measuring head (10', 10") is able to tilt around the placement point.

2. Measuring apparatus according to claim 1, wherein the measuring head (10', 10") has a sensor sleeve (11) in which the spacer (6, 6', 6") is integrated.

3. Measuring apparatus according to claim 1 or 2, wherein the measuring head (10', 10") is connected by an optical cable (2) to a light source (4) and to an evaluating and display device (5), and the handpiece (1) comprises a handle element (1.1) and a head part (1.2) having the spacer (6).

4. Measuring apparatus according to claim 3, wherein the measuring head (10', 10") is outfitted with a beam scanner which guides the measuring beam over the tooth surface in a predetermined measuring area.

5. Measuring apparatus according to one of claims 1 to 3, wherein the measuring system comprises direct-vision sensor optics (24) which are accommodated in the measuring head (10', 10").

6. Measuring apparatus according to one of claims 1 to 3, wherein the measuring system comprises sensor optics (24) which are accommodated in the measuring head (10', 10") and which have an angled beam path in an angular range of 90° to 135°.

7. Measuring apparatus according to claim 5 or 6, wherein the sensor optics (24) are designed to generate a measurement spot diameter between 2 µm and 10 µm and have a lateral resolution between 1 µm and 5 µm, a vertical resolution between 10 nm and 100 nm, and a working range between 300 µm and 1500 µm within a focal length range of the longitudinal chromatic aberration of the sensor optics (24).

8. Measuring apparatus according to claim 5 or 6, wherein the microoptics are designed as chromatic confocal sensor optics (24) and are optically connected to the light source and to a spectrometer via a beamsplitter, and the optical measuring system further comprises a control/evaluation/supply device (12) which is connected to a display device (14), the light source and the spectrometer.

9. Measuring apparatus according to claim 7, wherein the microoptics are designed as confocal sensor optics (24) and are optically connected by a beamsplitter to the light source and to an optical output measuring device which measures intensity for detecting back-reflected light, and the optical measuring system further comprises a control/evaluation/supply device (12) which is connected to a display device (14), the light source and the output measuring device.

10. Measuring apparatus according to claim 8, wherein, by means of the combination of the chromatic confocal sensor optics with the spacer, a chromatic confocal distance measurement can be converted to a vertical measurement of the reflection intensity by means of the spectrometer so that high carious activity can be identified at low reflection intensity and low carious activity can be identified at high reflection intensity.

11. Measuring apparatus according to claim 9, wherein, by means of a combination of a chromatic confocal sensor optics with the spacer, a chromatic confocal distance measurement can be converted to a vertical measurement of the reflection intensity by means of the output measuring device so that high carious activity can be identified at low reflection intensity and low carious activity can be identified at high reflection intensity.

12. Measuring apparatus according to claim 10 or 11, wherein a detection is provided as exclusive intensity measurement of reflected light of at least one wavelength which is sharply focused on the surface, wherein the reflection intensity is acquired so that a porosity or roughness of the enamel surface **characterized by** the depth profile can be determined.

13. Measuring apparatus according to claim 8, wherein the measuring head (10') is connected by an optical cable (2) via the beamsplitter to the light source and to the spectrometer, and the display device (14) is connected via a control cable (13) to the control/evaluation/supply device (12).

14. Measuring apparatus according to claim 13, wherein means are provided for attaching the display device (14) to a finger of a hand and a holder (15) is provided for temporarily holding the measuring head (10') on the display device (14).

15. Measuring apparatus according to one of claims 8 to 13, wherein the light source, the spectrometer and the control/evaluation/supply device (12) are constructed jointly as a PC card.

16. Measuring apparatus according to one of claims 8 to 13, wherein the light source, the spectrometer and the control/evaluation/supply device (12) are constructed jointly as an installed device in dental treatment chairs.

17. Use of a measuring apparatus according to one of claims 1 to 16 for differentiating tooth surface states which are **characterized by** erosive substance loss.

18. Use of a measuring apparatus according to one of claims 1 to 16 for assessing the polish quality of dental restorations.

19. Use of a measuring apparatus according to one of claims 1 to 16 for quantitative characterization of surface states of engineered surfaces for the purpose of determining a state-dependent material parameter.

## Revendications

1. Dispositif de mesure destiné à déterminer l'état d'activité de lésions carieuses initiales de l'émail des dents, ledit dispositif comprenant
- un système de mesure optique destiné à mesurer des pores dans l'émail des dents, ledit système comprenant une tête de mesure (10', 10"), qui est logée dans une poignée (1) et comprend un système de capteur optique (24) constitué d'un système microoptique passif compacte, destiné à la mesure de porosité et de rugosité, et
- un espaceur (6, 6', 6") destiné à être placé contre un point de contact sur la surface des dents, ledit espaceur (6, 6', 6") assurant une distance fixe prédéfinie entre des surfaces de sortie de faisceau et d'entrée de faisceau de la tête de mesure (10', 10") et la surface des dents ainsi que le basculement de la tête de mesure (10', 10") autour du point de contact.

2. Dispositif de mesure selon la revendication 1, dans lequel la tête de mesure (10', 10") comprend une gaine de capteur (11) dans laquelle l'espaceur (6, 6', 6") est intégré.

3. Dispositif de mesure selon la revendication 1 ou 2, dans lequel la tête de mesure est reliée à une source de lumière (4) et à un dispositif d'évaluation et d'affichage (5) par le biais d'un câble optique (2) et la poignée (1) est constituée d'un élément de poignée (1.1) et d'une partie supérieure (1.2) comprenant l'espaceur (6).

4. Dispositif de mesure selon la revendication 3, dans lequel la tête de mesure (10', 10") est équipée d'un scanner à faisceau guidant le faisceau de mesure sur la surface des dents dans une région de mesure prédéfinie.

5. Dispositif de mesure selon l'une quelconque des revendications 1 à 3, dans lequel le système de mesure comprend un système de capteur optique (24) à vision directe qui est logé dans la tête de mesure (10', 10").

6. Dispositif de mesure selon l'une quelconque des revendications 1 à 3, dans lequel le système de mesure comprend un système de capteur optique (24), qui est logé dans la tête de mesure (10', 10") et comprend un chemin optique formant un angle compris entre 90° et 135°.

7. Dispositif de mesure selon la revendication 5 ou 6, dans lequel le système de capteur optique (24) est destiné à produire un diamètre du point de mesure compris entre 2 µm et 10 µm et présente une résolution latérale compris entre 1 µm et 5 µm, une résolution verticale compris entre 10 nm et 100 nm, et une plage de travail dans une plage focale de l'aberration chromatique longitudinale du système de capteur optique (24) comprise entre 300 µm et 1500 µm.

8. Dispositif de mesure selon la revendication 5 ou 6, dans lequel le système microoptique se présente sous la forme d'un système de capteur optique (24) chromatique confocal et est relié optiquement à la source de lumière et à un spectromètre par le biais d'un séparateur de faisceau, le système de mesure optique comprenant en outre un dispositif de commande, d'évaluation et d'alimentation (12) qui est relié à un dispositif d'affichage (14), à la source de lumière et au spectromètre.

9. Dispositif de mesure selon la revendication 7, dans lequel le système microoptique se présente sous la forme d'un système de capteur optique (24) confocal et est relié optiquement, par le biais d'un séparateur de faisceau, à la source de lumière et à un dispositif de mesure de puissance destiné à mesurer l'intensité afin de mettre en évidence la lumière réfléchie, le système de mesure optique comprenant en outre un dispositif de commande, d'évaluation et d'alimentation (12) qui est relié à un dispositif d'affichage (14), à la source de lumière et au dispositif de mesure de puissance.

10. Dispositif de mesure selon la revendication 8, dans lequel la combinaison du système de capteur optique chromatique confocal et de l'espaceur permet la conversion d'une mesure de distance chromatique confocale dans une mesure perpendiculaire de l'intensité de réflexion au moyen du spectromètre, permettant ainsi la détection d'une activité carieuse élevée lorsque l'intensité de réflexion est basse et d'une activité carieuse basse lorsque l'intensité de réflexion est élevée.

11. Dispositif de mesure selon la revendication 9, dans lequel la combinaison d'un système de capteur optique chromatique confocal et de l'espaceur permet la conversion d'une mesure de distance chromatique confocale dans une mesure perpendiculaire de l'intensité de réflexion au moyen du dispositif de mesure de puissance, permettant ainsi la détection d'une activité carieuse élevée lorsque l'intensité de réflexion est basse et d'une activité carieuse basse lorsque l'intensité de réflexion est élevée.

12. Dispositif de mesure selon la revendication 10 ou 11, dans lequel on prévoit une détection comme seule mesure de l'intensité de la lumière réfléchie d'au moins une longueur d'onde nettement focalisée sur la surface et à laquelle l'intensité de réflexion est détectée, permettant ainsi de déterminer une porosité ou une rugosité de la surface de l'émail **caractérisée par** le profil en profondeur.

13. Dispositif de mesure selon la revendication 8, dans lequel la tête de mesure (10') est reliée à la source de lumière et au spectromètre par le biais du séparateur de faisceau, au moyen d'un câble optique (2'), et le dispositif d'affichage (14) est relié au dispositif de commande, d'évaluation et d'alimentation (12) par le biais d'un câble de commande (13).

14. Dispositif de mesure selon la revendication 13, dans lequel on prévoit des moyens pour fixer le dispositif d'affichage (14) à un doigt d'une main et un support (15) pour l'attachement temporaire de la tête de mesure (10') au dispositif d'affichage (14).

15. Dispositif de mesure selon l'une quelconque des revendications 8 à 13, dans lequel la source de lumière, le spectromètre et le dispositif de commande, d'évaluation et d'alimentation (12) constituent ensemble une carte PC.

16. Dispositif de mesure selon l'une quelconque des revendications 8 à 13, dans lequel la source de lumière, le spectromètre et le dispositif de commande, d'évaluation et d'alimentation (12) constituent ensemble un dispositif destiné à être intégré dans des fauteuils dentaires.

17. Utilisation d'un dispositif de mesure selon l'une quelconque des revendications 1 à 16 pour la différentiation entre des états superficiels des dents qui sont **caractérisés par** la perte érosive de substance.

18. Utilisation d'un dispositif de mesure selon l'une quelconque des revendications 1 à 16 pour l'évaluation de la qualité du poli des restaurations dentaires.

19. Utilisation d'un dispositif de mesure selon l'une quelconque des revendications 1 à 16 pour la caractérisation quantitative d'états superficiels dans le sens de la détermination d'un paramètre matériel qui dépend de l'état.
